# EUROPÄISCHE PATENTSCHRIFT

(11) **EP 0 531 777 B1**
(45) Veröffentlichungstag und Bekanntmachung des Hinweises auf die Patenterteilung: **12.02.1997**
(21) Anmeldenummer: 92114290.7
(22) Anmeldetag: 21.08.1992
(51) Int. Cl.: A61B 1/04, H04N 5/225

(54) **Videoendoskop mit Festkörperbildaufnahmevorrichtung**
Videoendoscope with solid state imaging apparatus
Vidéoendoscope avec dispositif d'imagerie à semi-conducteurs

(30) Priorität: 10.09.1991 DE 4129961
(43) Veröffentlichungstag der Anmeldung: 17.03.1993
(73) Patentinhaber: Richard Wolf GmbH, 75438 Knittlingen (DE)
(72) Erfinder: Wurster, Helmut, Dipl.-Ing., W-7519 Oberderdingen (DE)
(74) Vertreter: Wilcken, Thomas, Dipl.-Ing.

(56) Entgegenhaltungen:
- DE-A- 4 015 633
- US-A- 4 646 721
- US-A- 4 890 159

## Beschreibung

Die Erfindung geht aus von einem Videoendoskop gemäß dem Oberbegriff des Patentanspruchs 1. Ein derartiges Videoendoskop ist in der DE-A-40 15 633 beschrieben.

Videoendoskope der eingangs beschriebenen Art sind in verschiedenen Ausführungen im Gebrauch. So zeigt die US-A- 4 918 521 ein solches Instrument, bei dem der Halbleiterbildaufnehmer ein Festkörper-Bildaufnahmeelement umfaßt, dem ein optisches Linsensystem vorgeschaltet ist und das im wesentlichen rechtwinklig zu einem Schaltkreissubstrat angeordnet ist, so daß eine räumliche Anordnung von Festkörperaufnahmeelement, Substrat und elektronischen Bauelementen ermöglicht wird. Dabei werden diese Teile durch Vergießen zu einer Endoskopspitze geformt, die in ein Endoskop oder auch in ein Kameragehäuse einbaubar ist.

Diese Schrift zeigt eine Reihe von Ausführungsformen, die sich im wesentlichen durch die Anordnung und gegenseitige Zuordnung der genannten Elemente unterscheiden. Gemeinsames Merkmal ist dabei, daß die einzelnen Bauelemente auf dem mit Anschlüssen versehenen Schaltkreissubstrat aufgebracht und verdrahtet werden. Dabei ist nachteilig, daß die Einzelhybride relativ groß sind und ihre Verdrahtung schwierig und daher aufwendig ist.

Aus der DE-A-40 15 633 ist ebenfalls ein Videoendoskop entnehmbar, bei dem im distalen Endbereich ein Objektiv einem proximal folgenden Halbleiterbildaufnehmer und weiteren elektronischen Bauelementen vorgeordnet ist. Der Halbleiterbildaufnehmer und die weiteren elektronischen Bauelemente sind auf einem einstückigen keramischen Träger angeordnet, auf dem die Leiter zur Verbindung und zum Anschluß der elektronischen Bauelemente und des Halbleiterbildaufnehmers aufgebracht sind und der innerhalb des Endoskopschaftes sitzt und sich daran abstützt. Auf dem keramischen Träger ist noch ein Prisma zur Umlenkung des aus dem vorgeschalteten Objektiv gewonnen Bildes auf den Halbleiterbildaufnehmer angeordnet.

Auch aus der US-A- 4 809 680 ist ein Videoendoskop entnehmbar, welches die vorgenannten Bauelemente in der Endoskopspitze vereinigt, wobei diese jedoch als vorgefertigter Einsatz ausgebildet ist, in den das optische System mit den an diesem angeordneten elektronischen Bauelementen einsetzbar ist und welcher am Ende eines flexiblen Endoskopschaftes anbringbar ist.

Hingewiesen sei auch auf die Patentschriften US-A- 4 745 470, US-A- 4 832 003 und US-A- 4 757 805, aus denen ebenfalls Endoskopspitzen ähnlicher Ausführung entnehmbar sind.

Die in den zitierten Schriften offenbarten Konstruktionen weisen alle eine oder mehrere Platinen für den CCD-Chip und die übrige Elektronik auf, sowie eine damit verbundene Halterung für die Optik. Diese Elemente sind dann zu einem Endoskopkopf vergossen. Erst nach Abschluß dieses Gießverfahrens erhält dieser damit seine volle Stabilität, wobei auch bei diesen Ausführungen die aus dem Stand der Technik nach der US-A- 4 918 521 bekannten Nachteile vorliegen.

Es ist die Aufgabe der Erfindung, für den distalen Endbereich eines gattungsgemäßen Videoendoskops einen Aufbau anzugeben, der es ermöglicht, den Durchmesser gegenüber den bekannten Lösungen weiter zu verringern; gleichzeitig soll ein einfacherer Aufbau des Videoendoskopkopfes erreicht werden.

Diese Aufgabe wird durch die Merkmale des Patentanspruchs 1 gelöst.

Die mit dieser Ausführung erzielbaren Vorteile bestehen insbesondere darin, daß neben der Nutzung der an sich bekannten Vorteile der Keramikwerkstoffe - es sei beispielsweise auf das günstige Temperaturverhalten hingewiesen - das direkte Aufbringen der elektronischen Bauelemente, in erster Linie des CCD-Chips, eine Einsparung der meist keramischen Substratplättchen für die einzelnen Bauelemente möglich macht. Weiter kann auf die sonst üblichen Sockel verzichtet werden, und man gewinnt aufgrund des Fehlens der relativ viel Raum beanspruchenden Anschlußfähnchen der elektronischen Bauelemente zusätzlichen Raum, so daß der Durchmesser des Viedeoskops verringert werden kann bzw. bei sehr geringem Platzbedarf relativ große Flächen für die Unterbringung der übrigen Bauteile, Optik, Lichtleiter, Instrumenten-, Saug- und Spülkanäle, Elektronik und elektrische Verbindungen zur Verfügung steht.

In bevorzugter Ausgestaltung der Erfindung ist es zweckmäßig, wenn der Zentrierteil mit Durchbohrungen versehen ist, z.B. zur Aufnahme von Lichtleitern, zum Hindurchführen eines Hilfsinstrumentes, zur Führung von Spülflüssigkeit und zum Anbringen elektrischer Anschlüsse, da so eine komplette und exakte Vormontage des Videoendoskopkopfes ohne weitere Ausricht- und Haltehilfen ermöglicht wird. Um dabei die flexiblen Lichtleiter in bezug auf die Optik genau auszurichten kann vorgesehen sein, daß das Objektiv auf einem Keramikplättchen montiert ist, welches auf dem Tragteil aufbringbar ist und für die Aufnahme von Lichtleitern vorgesehene Durchbohrungen aufweist, die mit denjenigen in dem Zentrierteil fluchtend angeordnet sind.

Das erfindungsgemäße Videoendoskop ist nachstehend anhand einiger in der Zeichnung dargestellter Ausführungsbeispiele näher erläutert. Es zeigen:
- Figur 1: einen Längsschnitt durch einen erfindungsgemäßen Videoendoskopkopf,
- Figur 2: einen Schnitt entlang der Schnittlinie II-II in Figur 1,
- Figur 2a: einen Schnitt entlang der Schnittlinie IIa-IIa in Figur 1,
- Figur 3: einen Schnitt entlang der Schnittlinie III-III in Figur 1,
- Figur 4: eine andere Ausführungsform als in Darstellung gemäß Figur 3 mit einer Steckerleiste für den Anschluß der Signalleitungen und Elektronik,
- Figur 5: eine modifizierte Ausführung des Videoendoskopkopfes im Längsschnitt,
- Figur 6: einen Längsschnitt durch die Darstellung nach Figur 5 entlang der Schnittlinie VI-VI,
- Figur 7: eine weitere Variante eines Videoendoskopkopfes im Längsschnitt und
- Figur 8: einen Schnitt entlang der Schnittlinie VIII-VIII in Figur 7.

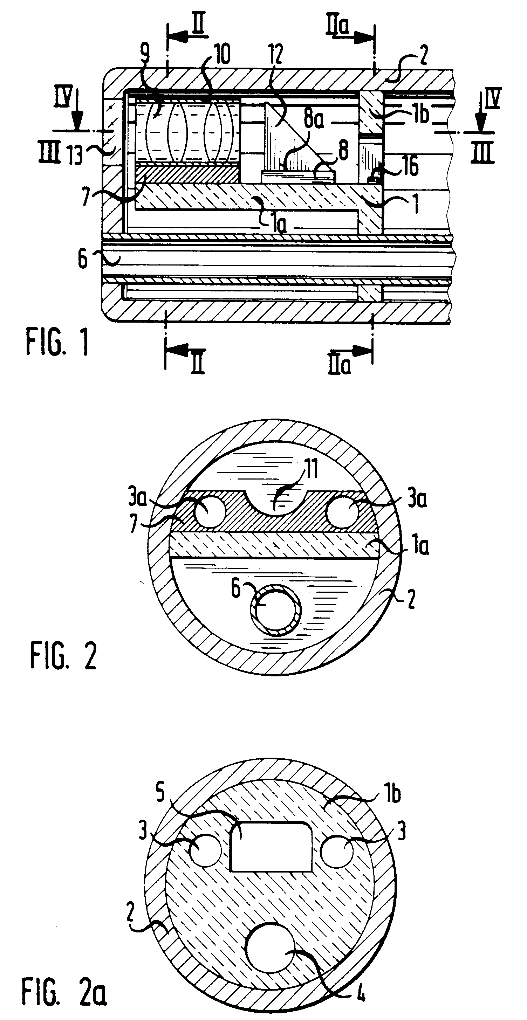

Die folgende Beschreibung bezieht sich zunächst auf die Ausführung gemäß den Figuren 1, 2, 2a und 3, die den distalen Endoskopkopf eines Videoendoskops zeigen. Wie ersichtlich umfaßt dieser Kopf einen Träger 1, der aus einem keramischen Werkstoff gefertigt ist. Dieser keramische Träger 1 weist die Form eines T auf. Ein sich in Richtung der Endoskopachse erstreckender Teil ist als Tragteil 1a ausgebildet und hat die Form eines Quaders. Hieran schließt im rechten Winkel ein Zentrierteil 1b an, der der Innenkontur des zylinderförmigen Endoskopschaftes 2 angepaßt ist. Der Zentrierteil 1b ist, wie in Figur 2a erkennbar, mit Durchbohrungen 3 und 4 sowie einer Ausnehmung 5 versehen. Durch die Bohrung 4 wird ein Hilfsinstrumentenkanal 6 hindurchgeführt. Die Bohrungen 3 dienen zur Durchführung der Lichtleiter durch den Zentrierteil 1b. Die Ausnehmung 5 wird für die Anbringung elektrischer Anschlüsse benötigt.

Wie weiter aus den Figuren 1 und 2 erkennbar ist, sind auf der Oberseite des Tragteils 1a ein vorzugsweise aus dem gleichen keramischen Werkstoff wie der Träger 1 gefertigtes Plättchen 7 und ein Festkörperbildaufnahmeelement (CCD) 8 angeordnet. Das Plättchen 7 dient als Tragteil für einen Teil der optischen Elemente, die von einem Metallzylinder 10 eingefaßt sind und das Endoskopobjektiv 9 bilden. Die Befestigung des Metallzylinders 10 erfolgt in einer Ausnehmung 11 des Plättchens 7. Das Plättchen 7 weist auch Durchbohrungen 3a auf, welche mit den Bohrungen 3 des Zentrierteils 1b fluchten und zur Durchführung der Lichtleiter dienen. Über dem Festkörperbildaufnahmeelement 8 ist in diesem Ausführungsbeispiel ein Prisma 12 angeordnet, das dazu dient, das durch das Objektiv 9 aufgenommene Bild auf das in diesem Beispiel mit seiner Aufnahmefläche 8a in einer zur Endoskoplängsachse parallelen Ebene angeordnete Festkörperbildaufnahmeelement 8 umzulenken. Im Bereich des Objektivs 9 ist das Endoskopende schließlich in üblicher Weise mit einem lichtdurchlässigen Abdeckplattchen 13 abgeschlossen.

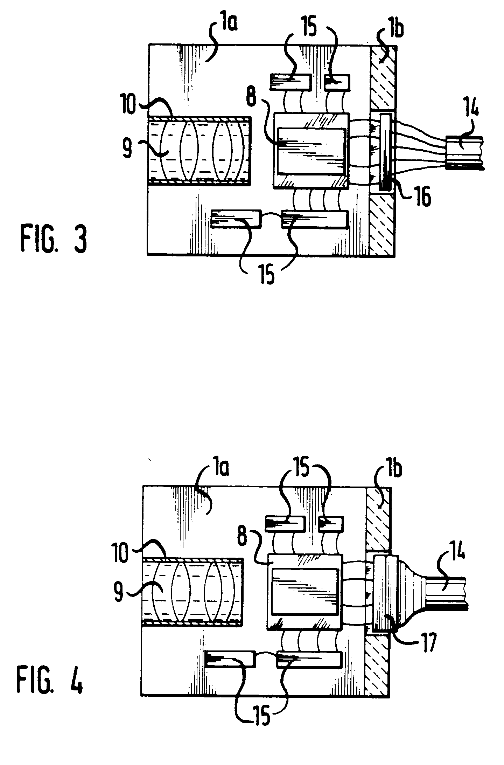

Der Beschaltung des Festkörperbildaufnahmeelementes 8 dienen elektronische Bauelemente 15, die sämtlich wie das Festkörperbildaufnahmeelement 8 selbst direkt auf die Keramik des Tragteils 1a aufgebracht sind. Die elektrische Verbindung der aufgebrachten elektronischen Bauelemente 8 bzw. 15 untereinander erfolgt in bekannter Weise z.B. durch Bonden oder durch aufgedampfte Leiterbahnen oder ähnliche Maßnahmen. Die Verbindung zwischen der auf dem Tragteil 1a befindlichen Elektronik mit den proximalwärts führenden Signalleitungen 14 wird gemäß Figur 3 mittels einer Lötleiste 16 hergestellt, wahrend diese Aufgabe bei der Ausfuhrung nach Figur 4 durch eine Steckerleiste 17 übernommen wird.

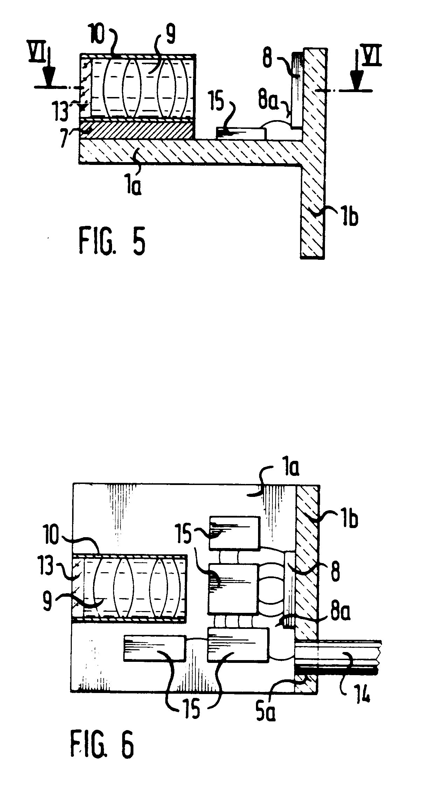

Bei der in den Figuren 5 und 6 dargestellten Ausführungsform des distalen Endoskopkopfes findet ein ebenfalls im wesentlichen T-förmiger Träger 1 Verwendung, auf dem das Endoskopobjektiv 9 sowie die elektronischen Bauelemente 15 fuhr die Beschaltung des Festkörperbildaufnahmeelementes 8 angeordnet sind. Diese Bauteile sind in der gleichen Weise wie vorstehend beschrieben aufgebracht. Der Unterschied zu dem Beispiel nach den Figuren 1 bis 4 besteht jedoch darin, daß das Festkörperbildaufnahmeelement 8 mit seiner Bildaufnahmefläche 8a in Richtung Objektiv weisend senkrecht zur optischen Achse des Objektivs 9 angeordnet ist. Daher ist hier auch der keramische Träger 1 dahingehend modifiziert, daß die Ausnehmung für die Kontaktierung zwischen Elektronik und Signalleitungen 14 nicht wie nach Figur 2a, sondern als seitliche Ausnehmung 5a, wie in Figur 6 zu sehen, angeordnet ist. Die Durchbohrungen 3 für die Lichtleiter werden selbstverständlich auch modifiziert angebracht, was jedoch nicht dargestellt ist. Wie bei dem vorausgehenden Ausführungsbeispiel werden auch in dem Beispiel nach den Figuren 5 und 6 die elektronischen Bauelemente direkt in der schon geschilderten Art und Weise auf den keramischen Träger 1 aufgebracht und, wie oben ebenfalls beschrieben, untereinander elektrisch verbunden. Die Verbindung zwischen Elektronik und Signalleitungen erfolgt in der seitlichen Ausnehmung 5a, und zwar wieder entweder über eine Löt- oder eine Steckerleiste.

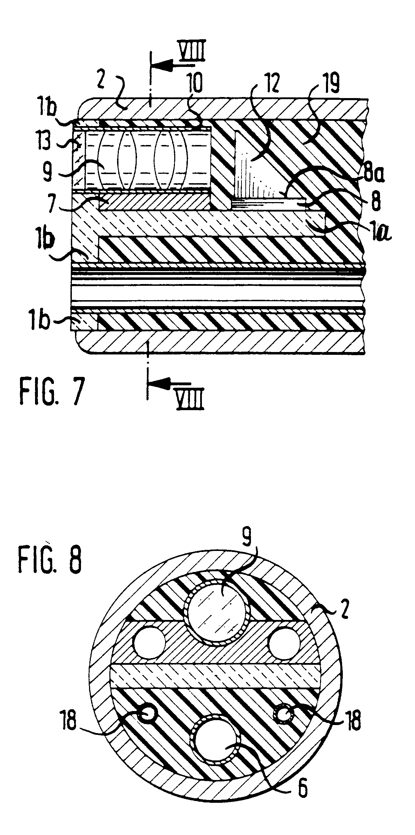

Bei der in den Figuren 7 und 8 dargestellten Ausführungsform des distalen Endoskopkopfes findet wieder ein keramischer Träger 1 von im wesentlichen T-förmiger Gestalt Verwendung, wobei jedoch der senkrecht zur Endoskopachse ausgerichtete Zentrierteil 1b den distalen Abschluß des Endoskops bildet und der quaderförmige Tragteil 1a in Richtung der Endoskopachse proximalwärts zeigt. Die Anordnung des Endoskopobjektivs 9 und der elektronischen Bauelemente 8 bzw. 15 erfolgt hier in der für die Ausführung nach den Figuren 1 bis 4 vorbeschriebenen Weise. Wie aus Figur 8 hervorgeht, sind zusätzlich zu den bereits erwähnten Kanälen zwei Spülkanäle 18 angeordnet, mit deren Hilfe das lichtdurchlässige Abdeckplättchen 13 vor dem distalen Ende des Ojektivs 9 von Verunreinigungen freispülbar ist, so daß stets ein optimales Bild erzielt werden kann.

Zweckmäßigerweise erfolgt eine Festlegung aller Bauteile, mindestens aber der elektronischen Bauelemente, durch Vergießen mittels eines lichtdurchlässigen Kunststoffs 19, wie das für alle Ausführungsbeispiele geltend in Figur 7 angedeutet ist. In weiterer Ausgestaltung kann die Breite des quaderförmigen Tragteils 1a des keramischen Trägers 1 auch kleiner sein als das lichte Innenmaß des Endoskopschaftes, was in den Figuren nicht gezeigt ist.

## Patentansprüche

1. Videoendoskop, in dessen distalem Endbereich ein Objektiv (9), ein diesem zugeordneter Halbleiterbildaufnehmer (8 bzw. 8a) und weitere elektronische Bauelemente (15) angeordnet sind, wobei der Halbleiterbildaufnehmer (8; 8a) und die weiteren elektronischen Bauelemente (15) auf einem einstückigen keramischen Träger (1) angeordnet sind, auf dem die Leiter zur Verbindung und/oder zum Anschluß der elektronischen Bauelemente (15) und des Halbleiterbildaufnehmers unmittelbar aufgebracht sind und der innerhalb des Endoskopschaftes (2) sitzt und von diesem abgestützt ist, dadurch gekennzeichnet, daß der einstückige keramische Träger (1) einen sich in Richtung des Endoskopschaftes (2) erstreckenden quaderförmigen Tragteil (1a) aufweist, der außer dem Halbleiterbildaufnehmer (8; 8a) und den elektronischen Bauelementen (15) zusätzlich das Objektiv (9) trägt, und daß sich stirnseitig an diesem Tragteil ein scheibenförmiger Zentrierteil (1b) anschließt, der sich im wesentlichen senkrecht zu der Längsausdehnung des Tragteils (1a) erstreckt und in seiner Außenkontur der Innenkontur des Endoskopschaftes (2) angepaßt ist.

2. Videoendoskop nach Anspruch 1, dadurch gekennzeichnet, daß der Zentrierteil (1b) mit Durchbohrungen (3,4,5,18) zur Aufnahme von Lichtleitern und/oder zum Hindurchführen eines Hilfsinstrumentes und/oder zur Führung von Spülflüssigkeit und/oder zum Anbringen elektrischer Anschlüsse versehen ist.

3. Videoendoskop nach Anspruch 1 oder 2, dadurch gekennzeichnet, daß das Objektiv (9) auf einem Keramikplättchen (7) montiert ist, welches auf dem Tragteil (1a) aufbringbar ist und für die Aufnahme von Lichtleitern vorgesehene Durchbohrungen (3a) aufweist, die zu denjenigen in dem Zentrierteil (1b) fluchtend angeordnet sind.

4. Videoendoskop nach Anspruch 1, 2 oder 3, dadurch gekennzeichnet, daß das Breitenmaß des quaderförmigen Trägerteils (1a) des keramischen Trägers (1) kleiner ist als das lichte Innenmaß des Endoskopschaftes (2).

## Claims

1. A video endoscope in whose distal end region there is arranged an objective (9), a solid state image sensor (8 or 8a) associated with said objective and further electronic components (15), said solid state image sensor (8,8a) and the further electronic components (15) being arranged on a one-piece ceramic carrier (1) on which the conductors for coupling and/or connecting the electronic components (15) and the solid state image sensor are directly deposited, and which is seated within the endoscope shank (2) and' is supported by said endoscope shank, characterized in that the one-piece ceramic carrier (1) comprises a cuboid carrying part (1a) which extends in the direction of the endoscope shank (2) and which, apart from the solid state image sensor (8 and 8a) and the electronic components (15), additionally carries the objective (9); and that a disc shaped centering part (1b) connects at the end face of this carrying part, said centering part extending essentially at right angles to the longitudinal extension of the carrying part (1a) and being adapted with its outer contour to the inner contour of the endoscope shank (2).

2. A video endoscope according to claim 1, characterized in that the centering part (1b) is provided with through holes (3,4,5,18) for receiving fibre optics and/or for guiding through an assisting instrument and/or for guiding flushing liquid and/or for attaching electrical connections.

3. A video endoscope according to claims 1 or 2, characterized in that the objective (9) is mounted on a ceramic lamina (7) which can be deposited on the carrying part (1a) and comprises through holes (3a) for receiving fibre optics, said through holes being arranged flush to those in the centering part (1b).

4. A video endoscope according to claims 1, 2 or 3, characterized in that the width measure of the cuboid carrying part (1a) is smaller than the clear inner measurement of the endoscope shank (2).

## Revendications

1. Vidéo-endoscope, dans la zone terminale distale duquel sont disposés un objectif (9), un appareil de prise de vue à semi-conducteurs (8, respectivement 8a) attribué à ce dernier et des composants électroniques supplémentaires (15), l'appareil de prise de vue à semi-conducteurs (8; 8a) et les composants électroniques supplémentaires (15) étant disposés sur un support céramique (1) en une seule pièce, sur lequel sont appliqués directement les conducteurs pour la liaison et/ou pour le raccord des composants électroniques (15) et de l'appareil de prise de vue à semi-conducteurs, et qui vient se loger à l'intérieur de l'arbre d'endoscope (2) tout en s'appuyant sur ce dernier, caractérisé en ce que le support céramique (1)en une seule pièce présente une partie de support (1a) de forme parallélépipédique s'étendant dans la direction de l'arbre d'endoscope (2), qui porte, en dehors de l'appareil de prise de vue à semi-conducteurs (8; 8a) et des composants électroniques (15), en outre l'objectif (9), et en ce qu'une partie dc centrage (1b) en forme de disque se raccorde du côté frontal à cette partie de support, qui s'étend essentiellement perpendiculairement à l'étendue longitudinale de la partie de support (1a) et qui est adaptée, quant à son profil externe, au profil interne de l'arbre d'endoscope (2).

2. Vidéo-endoscope selon la revendication 1, caractérisé en ce que la partie de centrage (1b) est munie d'alésages traversants (3, 4, 5, 18) pour que viennent s'y loger des conducteurs optiques et/ou pour le guidage à travers ces derniers d'un instrument auxiliaire et/ou pour le guidage d'un liquide de rinçage et/ou pour l'application de raccords électriques.

3. Vidéo-endoscope selon la revendication 1 ou 2, caractérisé en ce que l'objectif (9) est monté sur une plaquette céramique (7) qui peut être appliquée sur la partie de support (1a) et qui présente les alésages traversants (3a) prévus pour la réception de conducteurs optiques, qui sont disposés à fleur par rapport à ces derniers dans la partie de centrage (1b).

4. Vidéo-endoscope selon la revendication 1, 2 ou 3, caractérisé en ce que la mesure de largeur de la partie de support (1a) de forme parallélipipédique du support céramique (1) est inférieure à la mesure de diamètre interne de l'arbre d'endoscope (2).
